# EUROPEAN PATENT APPLICATION

(11) **EP 4 305 968 A1**
(43) Date of publication of application: **17.01.2024**
(21) Application number: 22767257.3
(22) Date of filing: 11.03.2022
(51) Int. Cl.: A23J 3/00, A23J 3/34, C12P 13/12, A23L 27/00, C12N 9/10, C12N 9/52, C12N 9/54, C12N 9/58, C12N 9/62, C12N 9/80, C12P 21/06, A23L 33/175, A23L 33/18, A23L 33/185

(54) **PROTEIN DEGRADATION PRODUCT PRODUCTION METHOD AND ENZYME PREPARATION**

(30) Priority: 11.03.2021 JP 2021038847; 10.08.2021 JP 2021130736
(71) Applicant: Amano Enzyme U.S.A. Co., Ltd., Elgin, Illinois 60124 (US); Amano Enzyme Inc., Nagoya-shi Aichi 460-8630 (JP)
(72) Inventor: OKUDA, Keita, Elgin, Illinois 60124 (US); HIURA, Keita, Kakamigahara-shi, Gifu 509-0109 (JP)
(74) Representative: Witthoff Jaekel Steinecke Patentanwälte PartG mbB
(86) International application number: PCT/JP2022/010765
(87) International publication number: WO 2022/191303

(57) **Abstract**

It is an object of the present invention to provide a practical means for generating a cysteine from a protein, which is suitable for utilization in the field of food products (food product use). According to the present invention, there is provided a method for producing a cysteine-containing proteolytic product, comprising; allowing a γ-glutamyl peptide hydrolase, a filamentous fungi-derived protease, and a bacterial protease to act on a protein material.

## Description

### Technical Field

The present invention relates to a method of generating a cysteine from a protein, an enzyme agent for production of a cysteine-containing proteolytic product, and intended use thereof.

### Background Art

Proteins contained in food products are important as nutrients, and because of palatability, animal proteins that are mainly contained in meats, seafoods, etc. have been preferred. In recent years, from the viewpoint of environmental protection and increasing health consciousness, a demand for plant proteins has been increasing as a substitute for animal proteins. However, in terms of palatability, plant proteins have had many problems, and thus, various studies have been conducted for the purpose of improving the physical properties and tastes of plant proteins. For example, Patent Document 1 discloses that, in a method for producing a soybean protein using transglutaminase and protease, the protease is used for the purpose of reducing a viscosity increased due to the transglutaminase. On the other hand, amino acids and peptides are important elements, not only as nutrients, but also as elements for the tastes and flavors of food products. For example, glutamic acid and aspartic acid give an *umami* taste and an acid taste; glycine, alanine, and threonine give sweetness; and tryptophan, isoleucine, and valine give bitterness. Therefore, studies regarding generation of amino acids or peptides from proteins have been conducted. Patent Document 2 discloses a method of generating low-molecular-weight peptides by allowing two or more types of enzymes having only endoprotease activity on soybean proteins. In addition, in order to impart a taste, a method of generating amino acids used as seasonings has also been studied. For example, Patent Document 3 discloses a method of obtaining an amino acid seasoning with a high glutamic acid content by hydrolyzing a protein raw material using a heat-resistant proteolytic enzyme and a heat-resistant glutaminase.

By the way, it has been known that a cysteine forms a Maillard reaction product and thereby imparts the flavor of a meat. Cysteine is mainly extracted from animal-derived raw materials (hairs and feathers), and addition of a cysteine to foods is not desirable. Patent Document 4 discloses a method of generating a cysteine by allowing acid protease and glutaminase to act on glutathione. However, the content of glutathione is different depending on the types of food products, and thus, situations in which glutathione can be utilized are limited.

Moreover, Patent Document 5 discloses that free aspartic acid and free glutamic acid are increased by treating soybean proteins with glutamic acid-specific endoprotease. Furthermore, Patent Document 6 discloses that, when soybean proteins are treated with subtilisin protease derived from *Bacillus licheniformis* (ALCALASE) or serine protease derived from *Nocardiopsis prasina* (SP1), an SP1 hydrolysate is less bitter than an ALCALASE hydrolysate.

### Prior Art Documents

### Patent Documents

Patent Document 1: JP Patent Publication No. 2006-141231 A
Patent Document 2: JP Patent Publication No. 5-252979 A (1993)
Patent Document 3: JP Patent Publication No. 48-82068 A (1973)
Patent Document 4: WO2021/002195
Patent Document 5: JP Patent Publication No. 2008-526261 A
Patent Document 6: JP Patent Publication No. 2011-530274 A

### Summary of Invention

### Object to be Solved by the Invention

It is an object of the present invention to provide a practical means for improving the rich taste or *umami* taste of a food product or the like.

### Means for Solving the Object

In order to achieve the aforementioned object, the present inventors have conducted intensive studies directed towards improving the rich taste or *umami* taste of a food product. As a result, the present inventors have found that a food product with an improved rich taste or *umami* taste can be produced by the combined use of a glutaminase that is one of γ-glutamyl peptide hydrolases, a filamentous fungi-derived protease, and a bacterial protease. The present invention has been completed by conducting further studies based on these findings. Specifically, the present invention provides the following aspects.

[1] A method for producing a proteolytic product, comprising;
   allowing a γ-glutamyl peptide hydrolase, a filamentous fungi-derived protease, and a bacterial protease to act on a protein material.
[2] The method according to [1], wherein the γ-glutamyl peptide hydrolase is a glutaminase, a γ-glutartransferase, or a γ-glutamylcyclotransferase.
[3] The method according to [1] or [2], wherein the γ-glutamyl peptide hydrolase is a glutaminase derived from Bacillus microorganisms.
[4] The method according to any one of [1] to [3], wherein the γ-glutamyl peptide hydrolase is a glutaminase derived from *Bacillus amyloliquefaciens.*
[5] The method according to any one of [1] to [4], wherein the filamentous fungi-derived protease is an acid protease derived from Aspergillus microorganisms or a neutral protease derived from Aspergillus microorganisms.
[6] The method according to any one of [1] to [5], wherein the filamentous fungi-derived protease is an acid protease derived from *Aspergillus oryzae,* a neutral protease derived from *Aspergillus oryzae,* or a neutral protease derived from *Aspergillus melleus.*
[7] The method according to any one of [1] to [6], wherein the bacterial protease is a protease derived from Bacillus or Geobacillus microorganisms.
[8] The method according to any one of [1] to [7], wherein the bacterial protease is a protease derived from *Geobacillus stearothermophilus.*
[9] The method according to any one of [1] to [8], wherein the protein material is an animal protein material, a plant protein material, or a microbial protein material.
[10] The method according to any one of [1] to [9], wherein the protein material is a plant protein material.
[11] The method according to any one of [1] to [9], wherein the protein material is a protein material derived from peas, soybeans, fava beans, chickpeas, barley, wheat, oats, rice, buckwheat, chives, millet, hemp, algae, almonds, cashew nuts, hazelnuts, pecan nuts, macadamia nuts, pistachios, walnuts, brazilnuts, peanuts, or coconuts.
[12] A food product, comprising the proteolytic product produced by the method according to any one of [1] to [11].
[13] An enzyme agent for production of a proteolytic product, comprising a γ-glutamyl peptide hydrolase, a filamentous fungi-derived protease, and a bacterial protease.
[14] The enzyme agent according to [13], wherein the γ-glutamyl peptide hydrolase is a glutaminase, a γ-glutartransferase, or a γ-glutamyl cyclotransferase.
[15] The enzyme agent according to [13] or [14], wherein the γ-glutamyl peptide hydrolase is a glutaminase derived from Bacillus microorganisms.
[16] The enzyme agent according to any one of [13] to [15], wherein the γ-glutamyl peptide hydrolase is a glutaminase derived from *Bacillus amyloliquefaciens.*
[17] The enzyme agent according to any one of [13] to [16], wherein the filamentous fungi-derived protease is an acid protease derived from Aspergillus microorganisms or a neutral protease derived from Aspergillus microorganisms.
[18] The enzyme agent according to any one of [13] to [17], wherein the filamentous fungi-derived protease is an acid protease derived from *Aspergillus oryzae,* a neutral protease derived from *Aspergillus oryzae,* or a neutral protease derived from *Aspergillus melleus.*
[19] The enzyme agent according to any one of [13] to [18], wherein the bacterial protease is a protease derived from Bacillus or Geobacillus microorganisms.
[20] The enzyme agent according to any one of [13] to [19], wherein the bacterial protease is a protease derived from *Geobacillus stearothermophilus.*

### Advantageous Effects of Invention

According to the present invention, a rich taste or an *umami* taste can be imparted to a food product and the like, or the rich taste or *umami* taste of a food product can be reinforced.

### Brief Description of Drawings

[Fig. 1] Fig. 1 shows the results of the evaluation of solidity in Example 2.
[Fig.2] Fig. 2 shows the results of the analysis of free amino acid in Example 2.
[Fig.3] Fig. 3 shows the results of the analysis of free amino acid in Example 3.
[Fig.4] Fig. 4 shows the results of the analysis of free amino acid in Example 3.
[Fig.5] Fig. 5 shows the results of the analysis of free amino acid in Example 3.
[Fig.6] Fig. 6 shows the results of the analysis of free amino acid in Example 3.

### Embodiments of Carrying out the Invention

According to the present invention, there is provided a method for producing a proteolytic product, comprising a step of allowing a γ-glutamyl peptide hydrolase, a filamentous fungi-derived protease, and a bacterial protease to act on a protein material.

According to the present invention, there is further provided an enzyme agent for production of a proteolytic product, the enzyme agent comprising a γ-glutamyl peptide hydrolase, a filamentous fungi-derived protease, and a bacterial protease.

The step of allowing a γ-glutamyl peptide hydrolase, a filamentous fungi-derived protease, and a bacterial protease to act on a protein material may be carried out in one step (i.e., a step of allowing the above three types of enzymes to simultaneously act on the protein material) or may also be carried out in two or three steps (i.e., a step of allowing one or two of the above three types of enzymes to act on the protein material, and a step(s) of allowing another/other enzyme(s) of the above three types of enzymes to act on the protein material).

Examples of the γ-glutamyl peptide hydrolase may include a glutaminase, a γ-glutartransferase, and a γ-glutamylcyclotransferase.
As such γ-glutamyl peptide hydrolase, a γ-glutamyl peptidase derived from, microorganism can be used, and examples thereof may include a glutaminase, a γ-glutamyltransferase, and a γ-glutamylcyclotransferase, which are derived from Bacillus microorganisms.

The γ-glutamyl peptide hydrolase is preferably a glutaminase derived from Bacillus microorganisms, and is more preferably a glutaminase derived from *Bacillus amyloliquefaciens* (for example, Glutaminase SD-C100S provided by Amano Enzyme Inc.).

The γ-glutamyl peptide hydrolase derived from microorganism may not be a purified product. For example, a culture solution, or a disrupted solution/an extract of microorganisms that generate the γ-glutamyl peptide hydrolase, or a partially purified product thereof, or the like, may be used. Also, γ-glutamyl peptide hydrolases derived from two or more types of microorganisms may be used in combination. Besides, γ-glutamyl peptide hydrolases derived from several types of microorganisms are commercially available (for example, the above-described Glutaminase SD-C100S), and these commercially available γ-glutamyl peptide hydrolases can be easily obtained and utilized.

Preferred examples of the filamentous fungi-derived protease may include an acid protease derived from Aspergillus microorganisms and a neutral protease derived from Aspergillus microorganisms.

Examples of the acid protease derived from Aspergillus microorganisms may include an acid protease derived *from Aspergillus oryzae* (for example, Protease M "Amano" SD and Protease HF "Amano" 150SD, which are provided by Amano Enzyme Inc.).

Examples of the neutral protease derived from Aspergillus microorganisms may include a neutral protease derived from *Aspergillus oryzae* and a neutral protease derived from *Aspergillus melleus.* Specific examples thereof may include a neutral protease derived from *Aspergillus oryzae* (PR-AX; product name: Proteax), a neutral protease derived from *Aspergillus oryzae* (PR-ASD; product name: Protease A "Amano" SD), a neutral protease derived from *Aspergillus melleus* (PR-P6SD; product name: Protease P "Amano" 6SD), and a neutral protease derived from *Aspergillus oryzae* (PR-AN100SD), which are provided by Amano Enzyme Inc.

The filamentous fungi-derived protease may not be a purified product. For example, a culture solution, or a disrupted solution/an extract of microorganisms that generate the filamentous fungi-derived protease, or a partially purified product thereof, or the like, may be used. Also, two or more types of filamentous fungi-derived proteases may be used in combination. Besides, several types of filamentous fungi-derived proteases are commercially available (for example, the above-described Protease M "Amano" SD and Protease HF "Amano" 150S, and also, Proteax, protease A "Amano" SD, protease P "Amano" 6SD, and PR-AN100SD), and these commercially available filamentous fungi-derived proteases can be easily obtained and utilized.

The bacterial protease is preferably a metalloprotease. With regard to the origin of the bacterial protease, the bacterial protease is preferably a protease derived from Bacillus or Geobacillus microorganisms, and is more preferably a *Geobacillus stearothermophilus-derived* protease. One example of the bacterial protease may be Thermoase PC10F provided by Amano Enzyme Inc.

The bacterial protease may not be a purified product. For example, a culture solution, or a disrupted solution/an extract of microorganisms that generate the bacterial protease, or a partially purified product thereof, or the like, may be used. Also, two or more types of bacterial proteases may be used in combination. Besides, several types of bacterial proteases are commercially available (for example, the above-described Thermoase PC10F), and these commercially available bacterial proteases can be easily obtained and utilized.

The enzyme agent of the present invention comprising a γ-glutamyl peptide hydrolase, a filamentous fungi-derived protease, and a bacterial protease may also comprise an excipient, a buffer agent, a suspending agent, a stabilizer, a preservative, an antiseptic, a normal saline and the like, as well as the active ingredients (i.e. the above-described 3 types of enzymes). Examples of the excipient that can be used herein may include lactose, sorbitol, D-mannitol, maltodextrin, and white sugar. Examples of the buffer agent that can be used herein may include phosphate, citrate, and acetate. Examples of the stabilizer that can be used herein may include propylene glycol and ascorbic acid. Examples of the preservative that can be used herein may include phenol, benzalkonium chloride, benzyl alcohol, chlorobutanol, and methylparaben. Examples of the antiseptic that can be used herein may include benzalkonium chloride, paraoxybenzoic acid, and chlorobutanol.

The content of the active ingredients (i.e. the above-described 3 types of enzymes) in the present enzyme agent is not particularly limited, and can be determined, as appropriate.

The enzyme agent of the present invention is generally provided in a solid state (for example, in the state of an immobilized enzyme formed by immobilizing an enzyme on a material capable of immobilizing the enzyme on the surface or inside thereof, such as granules, powders, silica or a porous polymer), or in a liquid state.

Conditions for allowing the γ-glutamyl peptide hydrolase, the filamentous fungi-derived protease, and the bacterial protease to act on the protein material include, for example, a reaction temperature of 15°C to 70°C, preferably 30°C to 65°C, and more preferably 40°C to 60°C.

The reaction time and the enzyme amount are not particularly limited, as long as the expected action can be exhibited. For example, the reaction time may be 5 minutes to 48 hours, preferably 10 minutes to 12 hours, and more preferably 15 minutes to 6 hours. The enzyme amount may be set to be such an amount that the concentration of each enzyme of the 3 types of enzymes contained in the reaction solution can be, for example, 0.001% (W/W) to 10% (W/W), and preferably 0.01% (W/W) to 2% (W/W).

The protein material is preferably an animal protein material, a plant protein material or a microbial protein material, and is more preferably a plant protein material. Specific examples of the protein material may include protein materials derived from peas, soybeans, fava beans, chickpeas, barley, wheat, oats, rice, buckwheat, chives, millet, hemp, algae, almonds, cashews, hazelnuts, pecan nuts, macadamia nuts, pistachios, walnuts, brazilnuts, peanuts, and coconuts.

According to the present invention, a proteolytic product containing a large amount of cysteine capable of forming a Maillard reaction (i.e. a cysteine-containing proteolytic product) can be produced. The cysteine capable of forming a Maillard reaction does not only include a cysteine that is in the state of a free amino acid, but also includes a peptide having a cysteine residue enabling a Maillard reaction. Among others, the cysteine that is in the state of a free amino acid is preferable.

According to the present invention, there is further provided a food product comprising a cysteine-containing proteolytic product that is produced by the method for producing a cysteine-containing proteolytic product according to the present invention.

The present invention will be specifically described in the following examples. However, these examples are not intended to limit the scope of the present invention.

### Examples

### < Example 1 >

The following experiment was carried out directed towards establishing a method of efficiently generating a cysteine from a protein.

### 1. Studies regarding generation of cysteine

### (1) Methods

Each type of protein material (12 g) was suspended in water to prepare a protein solution (15% (W/W), pH 5). Thereafter, 1.4 g of filamentous fungi-derived protease (Protease HF "Amano" 150SD, Amano Enzyme Inc.), 0.7 g of glutaminase (Glutaminase SD-C100S, Amano Enzyme Inc.), and 0.7 g of bacterial protease (Thermoase PC10F, Amano Enzyme Inc.) were dissolved in 20 mL of water to prepare an enzyme solution. After that, 1 mL of the enzyme solution was added to 30 mL of the protein solution at 50°C, followed by blending them, and the mixed solution was then treated at 50°C for 2 hours. Thereafter, the treated solution was centrifuged, and a supernatant was then recovered. Thiol groups of cysteine contained in the supernatant were quantified using a DTNB reagent. To 2.6 mL of a 0.1 M Tris buffer (1 mM EDTA, pH 7.05), 0.2 mL of a 25 mM DTNB solution (50 mM ammonium acetate, 1 mM EDTA, pH 5.0) and 0.2 mL of the supernatant diluted to an appropriate concentration were added, and they were then mixed with one another. Thereafter, the routine was left for 10 to 15 minutes, until the reaction has been completed. After completion of the reaction, the absorbance at 412 nm was immediately measured. The amount of cysteine residues in the supernatant was calculated from a calibration curve produced using a 10 µM to 50 µM cysteine solution (1 mM EDTA) instead of the supernatant. In addition, in order to confirm a change in the taste, 2 mL of the supernatant that had been neutralized with hydrochloric acid to pH 7 was prepared, and 1 mL of 20% glucose was then added to the prepared supernatant. The mixture was boiled for 30 minutes for a Maillard reaction, and the rich taste of the thus obtained reaction mixture was then examined. Specifically a change in the taste was examined by sensory evaluation with panelists.

### (2) Results

The release of a cysteine from all of the protein materials as a result of the enzyme treatment could be confirmed. Moreover, other than the use of a hemp protein, the improvement of a rich taste that is an important factor of a meat flavor could also be confirmed in the Maillard reaction products. Regarding the hemp protein, since the hemp-specific flavor was too strong, a change in the rich taste could not be confirmed.

**[Table 1]**

| Protein material | Product name | Protein content | Cysteine amount (µg/g raw material protein) | Change in rich taste after Maillard reaction |
|---|---|---|---|---|
| Pea protein | PURIS Pea 870 (PURIS) | 80% | 3.5 | Improved |
| Rice protein | Organic brown rice protein (Zen Principle) | 80% | 6.4 | Improved |
| Soybean protein | Soy Protein Isolate (NOW Sports) | 83% | 11.5 | Improved |
| Hemp protein | Hemp protein (Nutiva) | 50% | 13.8 | - |
| Almond protein | Almond Protein Powder (Noosh) | 60% | 6.8 | Improved |
| Oats powder | Whole Oat (Muscle Feast) | 18% | 33.9 | Improved |
| Algae protein | Whole Algal Protein (AlgaVia) | 63% | 15.3 | Improved |

### < Example 2 >

Water (105 g (1.5-times amount)) was added to 70 g of a soybean meat (soybean TVP (Textured Vegetable Protein) (Daizu Labo, soybean meat, minced type, Marukome Co., Ltd.), and the obtained mixture was then left for 10 minutes. Into the thus obtained mixture, 8.7 g of Metolose, 48.5 g of sunflower oil, and 26.7 g of Sun Rubber 10 (soy protein powders) were mixed, so that they became homogeneous. To 25 g of the above-obtained mixture, the enzyme solution shown in the following Table 2 was added in an amount of 1% (with respect to the soybean TVP), and they were then reacted at 50°C for 1 hour. After completion of the reaction, 6 mL of water was added to the reaction mixture to mold it, and the obtained mixture was then burned (in an oven at 110°C for 10 minutes). The obtained burned products were subjected to sensory evaluation (5-level evaluation of an *umami* taste and a flavor). The results of the sensory evaluation are shown in Table 3. "No enzyme" is used as a reference, and the greater the number, the more the improvement that could be shown. Thus, 1 indicates no changes, and 4 indicates a large improvement.

**[Table 2]**

| Table 2: Composition of enzyme agent: 50% filamentous fungi-derived protease (A) + 25% bacterial metalloprotease + 25% glutaminase (bacterial metalloprotease and glutaminase are the same as those used in Example 1) | |
|---|---|
| | (A) |
| 1 | *A. oryzae-*derived acid protease (PR-HF150SD) |
| 2 | *A. oryzae-*derived neutral protease (PR-AX) |
| 3 | *A. oryzae-*derived neutral protease (PR-ASD) |
| 4 | *A. melleus-*derived neutral protease (PR-P6SD) |
| 5 | *A. oryzae-*derived neutral protease (PR-AN100SD) |
| 6 | *A. oryzae-*derived acid protease (PR-MSD) |

**[Table 3]**

| Table 3: Results of sensory evaluation | | |
|---|---|---|
| | *Umami* taste | Roasting flavor |
| No enzyme | 1 | 1 |
| 1 | 2.5 | 2 |
| 2 | 3 | 3 |
| 3 | 3 | 3 |
| 4 | 3 | 3 |
| 5 | 3 | 4 |
| 6 | 2.5 | 2 |

The solidity of the obtained burned product was evaluated under the following conditions.
Apparatus used: Rheometer (COMPAC-100II)
Pushing rate: 60 mm/min
Movement distance of platform: 7 mm
(The solidity at a position 7 mm from the contact with the meat is measured.)

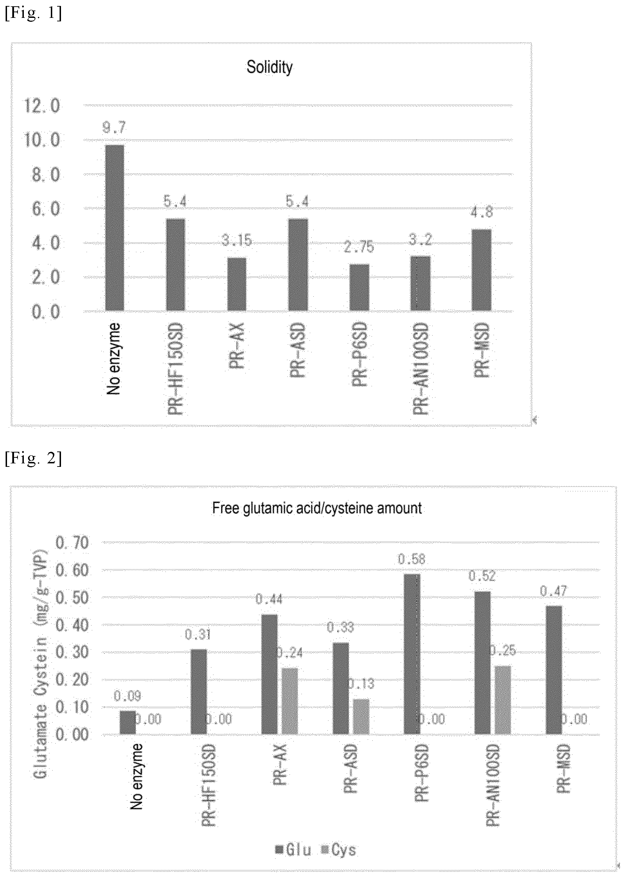

The results of the evaluation of the solidity are shown in Figure 1. The unit of the vertical axis in Figure 1 is N (Newton).

Free amino acid in the obtained burned product was analyzed as follows.

Distilled water (1 mL) was added to 1 g of a sample of the burned product, followed by blending them, and the obtained mixture was then centrifuged. The supernatant was mixed with ethanol at a ratio of supernatant : ethanol = 1 : 1 (the removal of the protein). The mixture was centrifuged, and a supernatant was then recovered. The recovered supernatant was 12.5-fold diluted with water, and thereafter, the diluted solution was filtered through a microfiltration membrane (MF), and was then analyzed by HPLC. The conditions for the HPLC analysis were as follows. The results of the free amino acid analysis are shown in Figure 2.

### Agilent HPLC (1260 InfinityII)

(A) buffer: 20 mM Na₂HPO_{4·}H₃PO₄, pH 8.2
(B) buffer: methanol : acetonitrile : water = 45 : 45 : 10
Column: HPH-C18, 2.7 µm, 3.0 × 100 mm (Poroshell)
Flow rate: 0.65 mL/min
0 to 0.35 min: A: 96%; B: 4%
0.35 to 13.4 min: A: 43%; B: 57%
13.4 to 13.5 min: A: 0%; B: 100%
13.5 to 15.7 min: A: 0%; B: 100%
15.7 to 15.8 min: A: 96%; B: 4%
15.8 to 18 min: A: 96%; B: 4%

### < Example 3 >

Enzymes were added to a 10% (w/v) plant protein solution (peas: Pea Protein (Usuki Pharmaceutical Co., Ltd.); soybeans: Sun Rubber 10 (FUJI OIL CO., LTD.)) in an amount of 4%, with respect to the weight of the plant proteins (2% filamentous fungi-derived protease + 1% bacterial protease + 1% glutaminase). The obtained mixture was treated at 50°C for 2 hours at 400 rpm for reaction. The reaction product was boiled for 10 minutes, and was then centrifuged to recover a supernatant. The obtained sample supernatant was subjected to sensory evaluation and free amino acid analysis.

### (Sensory evaluation)

The sample supernatant (4 mL) was collected, and 2 mL of 20% glucose was added to the sample supernatant. The obtained mixture was added into boiled water, and was treated for 30 minutes, and thereafter, sensory evaluation was carried out. The results are shown in Table 4. "No enzyme" is used as a reference, and the greater the number, the more the improvement that could be shown. Thus, 1 indicates no changes, and 5 indicates a large improvement.

**[Table 4]**

| Raw material | Enzyme | Taste | Flavor | Numerical value of *umami* taste |
|---|---|---|---|---|
| Peas | None | Neither *umami* taste nor rich taste | Sweet flavor | 1 |
| | 1 | A little *umami* taste and a little rich taste | Roasting flavor | 2 |
| | 2 | An *umami* taste and a rich taste | Roasting flavor | 3 |
| | 3 | An *umami* taste and a rich taste | Roasting flavor | 3 |
| | 4 | An *umami* taste and a rich taste | Roasting flavor | 3 |
| | 5 | An *umami* taste and a rich taste | Roasting flavor | 3 |
| | 6 | A little umami taste and a little rich taste | Roasting flavor | 2 |
| Soybeans | None | Neither *umami* taste nor rich taste | Sweet flavor | 1 |
| | 1 | A little *umami* taste | Roasting flavor | 2 |
| | 2 | Strong *umami* taste | Roasting flavor | 3 |
| | 3 | Strong *umami* taste | Roasting flavor | 3 |
| | 4 | Strong *umami* taste | Roasting flavor | 3 |
| | 5 | Strong *umami* taste | Roasting flavor | 4 |
| | 6 | A little *umami* taste | Roasting flavor | 2 |

### (Free amino acid analysis)

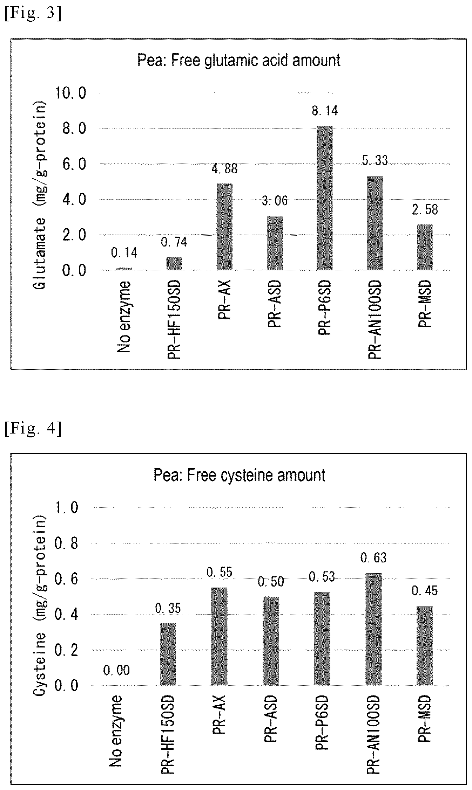

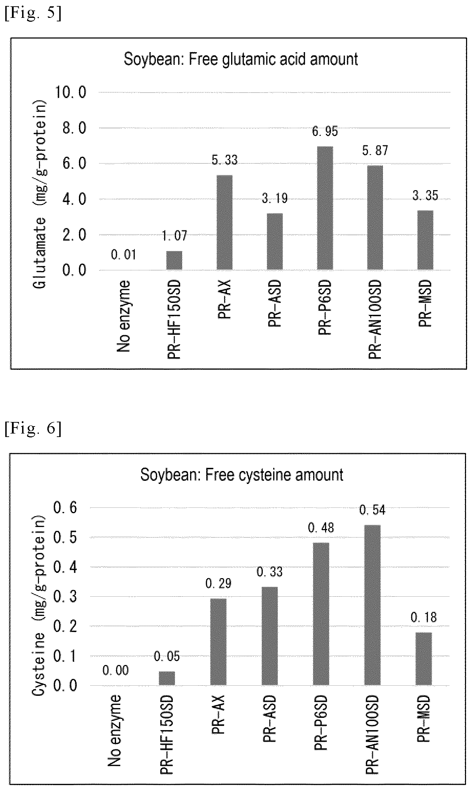

The sample supernatant was mixed with ethanol at a ratio of sample supernatant : ethanol =1:1 (the removal of the protein). The mixture was centrifuged, and a supernatant was then recovered. The recovered supernatant was 12.5-fold diluted with water, and thereafter, the diluted solution was filtered through a microfiltration membrane (MF), and was then subjected to HPLC analysis (amino acid analysis) under the same conditions as those applied in Example 2. The results of the free amino acid analysis are shown in Figure 3 to Figure 6.

## Claims

1. A method for producing a cysteine-containing proteolytic product, comprising;
allowing a γ-glutamyl peptide hydrolase, a filamentous fungi-derived protease, and a bacterial protease to act on a protein material.

2. The method according to claim 1, wherein the γ-glutamyl peptide hydrolase is a glutaminase, a γ-glutartransferase, or a γ-glutamylcyclotransferase.

3. The method according to claim 1 or 2, wherein the γ-glutamyl peptide hydrolase is a glutaminase derived from Bacillus microorganisms.

4. The method according to any one of claims 1 to 3, wherein the γ-glutamyl peptide hydrolase is a glutaminase derived from *Bacillus amyloliquefaciens.*

5. The method according to any one of claims 1 to 4, wherein the filamentous fungi-derived protease is an acid protease derived from Aspergillus microorganisms or a neutral protease derived from Aspergillus microorganisms.

6. The method according to any one of claims 1 to 5, wherein the filamentous fungi-derived protease is an acid protease derived from *Aspergillus oryzae,* a neutral protease derived from *Aspergillus oryzae,* or a neutral protease derived from *Aspergillus melleus.*

7. The method according to any one of claims 1 to 6, wherein the protein material is an animal protein material, a plant protein material, or a microbial protein material.

8. The method according to any one of claims 1 to 7, wherein the protein material is a plant protein material.

9. The method according to any one of claims 1 to 7, wherein the protein material is a protein material derived from peas, soybeans, fava beans, chickpeas, barley, wheat, oats, rice, buckwheat, chives, millet, hemp, algae, almonds, cashew nuts, hazelnuts, pecan nuts, macadamia nuts, pistachios, walnuts, brazilnuts, peanuts, or coconuts.

10. A food product, comprising the cysteine-containing proteolytic product produced by the method according to any one of claims 1 to 9.

11. An enzyme agent for production of a cysteine-containing proteolytic product, comprising a γ-glutamyl peptide hydrolase, a filamentous fungi-derived protease, and a bacterial protease.

12. The enzyme agent according to claim 11, wherein the γ-glutamyl peptide hydrolase is a glutaminase, a γ-glutartransferase, or a γ-glutamylcyclotransferase.

13. The enzyme agent according to claim 11 or 12, wherein the γ-glutamyl peptide hydrolase is a glutaminase derived from Bacillus microorganisms.

14. The enzyme agent according to any one of claims 11 to 13, wherein the γ-glutamyl peptide hydrolase is a glutaminase derived from *Bacillus amyloliquefaciens.*

15. The enzyme agent according to any one of claims 11 to 14, wherein the filamentous fungi-derived protease is an acid protease derived from Aspergillus microorganisms or a neutral protease derived from Aspergillus microorganisms.

16. The enzyme agent according to any one of claims 11 to 15, wherein the filamentous fungi-derived protease is an acid protease derived from *Aspergillus oryzae,* a neutral protease derived from *Aspergillus oryzae,* or a neutral protease derived from *Aspergillus melleus.*
